# EUROPEAN PATENT APPLICATION

(11) **EP 1 110 516 A1**
(43) Date of publication of application: **27.06.2001**
(21) Application number: 99125932.6
(22) Date of filing: 23.12.1999
(51) Int. Cl.: A61F 5/443

(54) **Hygiene article comprising a membrane containing interface device and body adhesives**

(71) Applicant: THE PROCTER & GAMBLE COMPANY, Cincinnati, Ohio 45202 (US)
(72) Inventor: Schmidt, Matthias, 65510 Idstein (DE); Ehrnsperger, Bruno Johannes, 65936 Frankfurt (DE)
(74) Representative: Kohol, Sonia

(57) **Abstract**

The present invention is a hygiene article comprising a liquid handling member with a membrane assembly having a porous membrane, which separates a first zone which comprises a liquid receiving region from a second one, which is connected to a suction device source, whereby the membrane assembly is capable of maintaining a pressure differential between the second zone and the first zone without permitting air to penetrate from the first zone to the second zone, wherein this article further comprises a fixation means of the body adhesive type to hold the liquid receiving region in registry with liquid releasing body openings of the wearer during its intended use.

## Description

The present invention relates to hygiene articles, in particular to articles intended to receive bodily liquids, such as urine in case of absorbent articles like diapers or external or internal catheter, or menses or blood, such as for feminine protection articles or wound treating articles, sweat, saliva.

### Background / problem

Hygienic articles comprising membranes are known from the prior art, such as disclosed in US-A5.678564 or PCT application US 98/13497.

Whilst such articles provide significantly improved liquid handling performance, there is still a need to provide sealing between the perimeter of the article and the skin of the wearer. Thus, US-A-5.678.564 discloses a preferred embodiment wherein the perimeter of the article is treated with petrolatum jelly as a sealing agent, so as to prevent liquid leaking outside of the article.

In addition to the sealing, it is important that the article remains affixed in ascertain position on the wearer to maintain the relative positioning of the article with the liquid releasing body openings of the wearer. For this purpose, in US-A-5.678.564, a pant style assembly is suggested.

However, there is still a need to improve on the functionality, simplicity during use, and application, of such articles.

### Summary

The present invention is a hygiene article comprising a liquid handling member with a membrane assembly having a porous membrane, which separates an first zone which comprises a liquid receiving region from a second one, which is connected to a suction device source, whereby the membrane assembly is capable of maintaining a pressure differential between the second zone and the first zone without permitting air to penetrate from the first zone to the second zone, wherein this article further comprises a fixation means of the body adhesive type to hold the liquid receiving region in registry with liquid releasing body openings of the wearer during its intended use.

Preferably, the adhesive exhibits a skin adhesion value of at least 0.1 N/cm, preferably 0.5 N/cm. It is further preferred, that the body adhesive has a skin adhesion value of not more than 7 N/cm, preferably of not more than 5 N/cm. A particular embodiment is of the hydrogel type.

The hygiene article can have an interface device comprising the first zone with the liquid receiving regions, the membrane assembly and the second zone, and the article can have a liquid storage device. The body adhesive can be used to attach either the interface device, or the storage device to the body of the wearer.

Whilst the interface device needs to be in proximity of the body releasing openings, the liquid storage device can be located remotely, and can be connected to the interface device by a drain tube.

The body adhesive can be the only attachment means of the article to the wearer, or there can be further fixation means.

### Detailed description

The absorbent article of the present invention can be worn by a wearer such that the wearer retains his mobility during use of the article of the present invention. In some embodiments of the present invention, the absorbent article of the present invention comprises an attachment means which is unitary with the article. The term "unitary" as used herein indicates that the attachment means is joined to the absorbent article. Alternatively, the absorbent article of the present invention may be attached to the lower torso of the wearer by an attachment means which is not unitary with the article such as for example a pant.

The absorbent article may be a system comprising two connected elements, such as an interface device connected to a storage device. During use, the interface device needs to be close to the exudate releasing body openings, such as the urethra, whilst the liquid storage device may be next to it - i.e. also in the region of the lower torso of the wearer - or may be located more remotely by being connected such as via a drain tube as described in US-A-5.678.564. The remote location may be on the wearer, such as in the thigh region of the legs, or may be connected to a device like a bed or a wheel-chair, if this were more appropriate for the wearer, and or for the design of the storage device, i.e. if this is a relatively small liquid receiving bag or a larger device like a vacuum surge pump.

A liquid removal member in the present context is considered to be a system, wherein a liquid is penetrating through a membrane or a membrane assembly comprising such a membrane by means of a potential difference, such as pressure differential like a suction or a vaccum. Whilst such a liquid can be of almost any type, the following description will describe the mechanisms by using aqueous liquids, such as salt solutions like urine. It will be apparent to the skilled person, to re-apply the teaching for other liquids, such as oily substances as have been discussed in more detail in PCT applications US 99/14644 or US 99/14645.

Such systems function by the principle that certain porous membranes under certain conditions can be permeable to liquids, but not to gases like air, as long as the "potential differential " such as the pressure differential between the two sides of such a membrane does not exceed a certain value characteristic for the material and the liquid within the pores of the material - the bubble point pressure. This latter is often expressed in "height of water column" which corresponds to the pressure exerted by such a column on the material under normal gravity conditions.

For aqueous liquids, the material for the membrane is preferably hydrophilic and has a pore size on the order of about 5 to about 30 µm, more preferably about 10 to 20 µm. Once the membrane has been wetted it will support a suction pressure typically corresponding to about 12.5 to about 150 cm column of water without permitting air to pass. Thus, if suction is applied to a first side of a wetted membrane, liquid contacting the membrane on the other side will be drawn by the suction through the membrane to the first side of the membrane, from where it can further be removed, for example by being sucked by means of a vaccum through a drain tube to a reservoir. As long as the filter material or membrane remains wet, air does not pass through the filter and suction is maintained without active pumping. If too much vacuum is applied to the membrane there is a risk that the bubble point of the membrane will be surpassed, thereby allowing air or gas to penetrate through, which can lead to a loss of the vacuum, and of the liquid handling functionality. Thus the amount of vacuum should approach as close as possible but not exceed the bubble point pressure.

In such a system, the membrane needs to be "hermetically sealed" to the other elements, which means that a gas (and especially air) can neither pass from the outside environment to the inside of the member, when the membrane is saturated with liquid, as long as the pressure differential does not exceed the bubble point pressure.

It is further generally desirable for the absorbent article of the present invention that it is comfortable for the wearer, whereby this has to be met by interface device and also by the liquid receiving device if this is worn by the user.

This requirement for comfort relates to properties and designs of the absorbent article as such, such as its size, weight, shape and flexibility to conform to shape and movements of the wearer, but it also has significant requirements for the fixation of the article on the wearer.

Generally, attachment means for fixing an absorbent article on the wearer during its intended use are well known in the art.

As described in EP-A-0.873.739, which is included herein by reference, these attachment means can be grouped into "mechanical" types and body adhesive types. The first group comprise the conventional application and fixation by means of adhesively connecting the front and rear parts of an article around the waist - thereby creating a tension line along a belt shaped part of the article, which in turn creates friction between the article and the wearer. Instead of the adhesive tapes, mechanical fastening systems of the "hook and loop" type can be used. Also into the first group belong the "pant fixation" means, whereby either an integral article can be used (such as with the so called "Pull on" diapers), or a separate pant (such as a stretch pant) can be combined with an absorbent pad, or an absorbent pad can be combined with regular underwear. For these options, there can be additional fixation means between the pants and the pads, such as well known "panty fastening adhesives" to releasably fix pad in panties during use.

In contrast to these fixations mechanism, the "body adhesive" systems rely on adhesion mechanism between the article and the skin of the wearer. Such "topical" application of the article is described in the above mentioned application EP-A-0.873.739.

For articles according to the present invention, it is important, that the membranes of the article are kept in alignment with the liquid releasing body openings. Whilst in some applications it can be desirable to seal the article periphery with the skin of the wearer to minimize the risk of liquid leaking therethrough, the body adhesives of the present invention rather aim at mechanically supporting the article in the appropriate position. Hence, the adhesive must enable forces to be transmitted to carry the weight of the article, such as gravitational forces, or forces resulting from movement of the wearer. There can be a wide range of the amount of transmitted forces, depending on the particular design of the article.

For example, when the article has a liquid storage member, which is remote from the liquid receiving member, the weight of the latter can be significantly reduced, and hence the requirements for the body adhesive width regard to forces which it can transmit, can be reduced.

Similarly, embodiments, wherein the liquid transport member is prefilled with a liquid so as to not require any activation, will put a higher requirement to the adhesion forces for the pre-loading phase than embodiments where the member is activated by the receiving liquid. Generally, whilst it is desired to design such articles having a low overall weight (such as described in more detail in co-pending application "Liquid removal system having reduced dimensions and reduced weight" Attorney docket CM-2255), weights of such systems can be as much as 200 g or even up to 500 g. As described in the same reference, it also will be desired to minimize the dimensions of the article, or at least of the liquid receiving device, such that the local stress as applied to the skin can be quite substantial.

At the same time, there are other user specific requirements, such as more or less skin sensitivity because of age (babies versus elderly people), wearing time (permanent, part-time, change frequency). Generally, it is desirable to have as little occlusion effect of the article as possible, and preferred "breathable" adhesive formulations allow gases such as air or water vapor to permeate through. Even further, particular adhesion properties can be linked to particular shape for particular applications (like gender specific articles, or articles for urinary incontinence only, compared to articles to receive urine and feces). Thus, a particularly important property of a body adhesive is the balancing of adhesion to the skin with allowing a removal with as little pain as possible.

Henceforth, a suitable adhesive should have a certain peel strength. A suitable method to assess this property is the "Peel adhesion method", as described in detail in the "Test method section" of European Patent application 99102040, which is incorporated herein by reference, whereby the adhesive is applied by means of a suitable substrate to human skin, and then peeled off under controlled conditions. Suitable materials exhibit a peel strength value of at least 0.1 N/cm, preferably of at least 0.5 N/cm, and for higher weights, respectively smaller application areas for the glue have 3.0 N/cm or more. However, the peel strength values should not be excessively high, as otherwise it will be too painful to remove the adhesive from the skin, and peel strength values of more than about 7.0 N/cm or more preferably of more than 5.0 N/cm should be avoided. Thereby, it will be desirable to have sufficient cohesion of the adhesive balanced with the application thickness of the adhesive to have sufficient integrity of the adhesive layer to not tear during removal, which otherwise could leave undesirable residues on the skin. As described in the same application, preferred adhesive formulations exhibit certain rheological properties, namely the elastic modulus G' and the viscous modulus G", which can be evaluated at 37°C, and which should be evaluated at different dynamic frequencies to replicate various in-use conditions, such as between a lower frequency of about 1 rad/s to simulate applications typically executed at a lower peel rate (or speed) such as application of an article having an adhesive, and a higher frequency of about 1/100 rad/s, to simulate the faster removal of the article and/or the adhesive.

There are various approaches to arrive at compositions for suitable body adhesives. Generally, such compositions include a polymeric component, such as block-copolymers, such as SBS, SBR, or SEBS, or cross-linked di-methyl-acrylates, a plasticizer, such as water or glycerin in case of hydrophilic formulations or oil in case of hydrophobic formulations, or a mixture thereof for mixed phase compositions, and a tackifyer, such as polymeric resins to adjust the Glass transition temperature. Further additives can be added as well known in the art, such as preservatives, antioxidants, anti-UV decomposition agents, pigments, mineral filler etc.

Such formulations can be tailored to provide hydro-colloid compositions, such as described in more detail in EP-A-0.753.290, or GB-A-2.116.849, both of which publications being incorporated herein by reference, which typically have small particle size elements suspended in an overall liquid phase, and which typically show good adhesion properties, relatively high stiffness, and tend to show relatively high pain feel levels.

Such formulations can also be tailored to provide oil-gel compositions, such as described in detail in PCT publication 98/27909, which is incorporated herein by reference, or to provide - as currently most preferred compositions - hydrogel formulations, as described in EP-A-0.850.649, which is also incorporated herein by reference, or in the above mentioned EP application 99102040. Following the description of the latter application, compositions can be readily formulated to balance a water sensitivity with a wet peel strength, such that the adhesive provides sufficient peel strength when applied to dry or wetted skin, however, becomes soluble upon excess liquid.

In order to satisfy the general requirements for the articles as described in the above, the adhesives can be applied to the perimeter of the article. In a particular embodiment, this perimeter can be designed to form a "flange" to minimize gapping between the article and the body of the wearer. Preferably, this flange is flexible so as to conform to the curvature of various wearers, or to the changing curvature of one and the same wearer (such as when changing position from standing to sitting or laying). This flexibility can range from generally shape maintaining - such as described in US-A-5.678..564 - or it can be generally pliable such as when made form rubbery materials, such as further disclosed in above mentioned EP application 99102040. It also can include resilient materials, such as foams or wadding.

The above described perimeter of the article, or flange, where applicable, can be of relatively small extension, or can be wide. Accordingly, the adhesive can be applied along a thin line such as of a width of 5 mm or less, or cover a substantial area such as by having a width of more than 15 mm. The adhesive can also be applied to particular attachment extensions of the article or of the flange thereof, such as in the form of wings, or ears, or attachment stripes.

The adhesive can be applied essentially to the complete perimeter or flange of the article in a continuous form, or it can be applied in a non-continuous pattern, such as in a plurality of dots , or short stripes, or the like.

The adhesive can also be applied to other parts of the article not on the perimeter thereof, but in the interior surface. Provided, that the adhesive does not impede the liquid handling functionality, it also can be applied on the membrane. In a particular embodiment, the adhesive can form a dissolvable layer on the surface of the membrane, which readily dissolves upon being contacted with the receiving liquid, but which remains adhesively functional in non-wetted parts or in the parts only wetted to a lower degree, i.e. not with excess liquid, such as by sweating or surface wetting as a result of wetted membrane pores.

Whilst the above explanation uses primarily hygiene applications such as incontinence articles, like external catheter, this should in no way limit the present invention to such applications. The skilled person will readily apply the principles to other hygiene articles such as internal catheter, or bandages, or wound dressings. In particular, during surgeries, often the need arises to capture relatively large amounts of body fluids in places which are difficult to reach otherwise.

## Claims

1. A hygiene article comprising a liquid handling member
with a membrane assembly having a porous membrane, which separates a first zone comprising a liquid receiving region from a second zone, which is connected to a suction device source, whereby said membrane is capable of maintaining a pressure differential between said second zone and said first zone without permitting air to penetrate from said first zone to said second zone,
characterized in that
said hygiene article further comprises a fixation means of the body adhesive type to hold the liquid receiving region in registry with liquid releasing body openings of the wearer during its intended use.

2. A hygiene article according to claim 1, wherein
said body adhesive has a skin adhesion value of at least 0.1 N/cm, preferably 0.5 N/cm.

3. A hygiene article according to claim 1 or 2, wherein
said body adhesive has a skin adhesion value of not more than 7 N/cm, preferably not more than 5 N/cm.

4. A hygiene article according to any of claims 1 to 3, wherein said body adhesive is selected from the group of oil-gels, hydro-colloids and hydrogels.

5. A hygiene article according to any of the preceding claims, further comprising a liquid storage device connected to said second zone by a drain tube.

6. A hygiene article according to claim 5, wherein said liquid storage device comprises body adhesive for attachment to the body of the wearer.

7. A hygiene article according to claim 5, wherein said liquid storage device is not located in the proximity of the lower torso of the wearer.

8. A hygiene article according to any of the preceding claims, wherein said first zone, said second zone and said membrane assembly form an interface device hermetically sealed and connected to said suction device,

9. A hygiene article according to claim 5, wherein said interface device comprises body adhesive for attachment to the body of the wearer.

10. A hygienic article according to any of the preceding claims, further comprising a secondary mechanical fixation means.
